(19) Europäisches Patentamt
European Patent Office
Office européen des brevets

(11) Veröffentlichungsnummer: **0 278 211 B1**

(12) # EUROPÄISCHE PATENTSCHRIFT

(45) Veröffentlichungstag der Patentschrift: **01.08.90**

(51) Int. Cl.[5]: **C07C 37/16**, C07C 39/367, C07C 39/15, B01J 29/08

(21) Anmeldenummer: **88100035.0**

(22) Anmeldetag: **05.01.88**

(54) **Verfahren zur Herstellung von p-substituierten o-Benzylphenolen.**

(30) Priorität: **14.01.87 DE 3700917**

(43) Veröffentlichungstag der Anmeldung: **17.08.88 Patentblatt 88/33**

(45) Bekanntmachung des Hinweises auf die Patenterteilung: **01.08.90 Patentblatt 90/31**

(84) Benannte Vertragsstaaten: **CH DE FR GB IT LI**

(56) Entgegenhaltungen:
**EP-A- 0 029 333**
**DE-A- 2 713 565**
**GB-A- 1 150 209**
**US-A- 4 514 577**

**CHEMICAL ABSTRACTS, Band 93, Nr. 7, 18. August 1980, Seite 922, Zusammenfassungsnr. 71184u, Columbus, Ohio, US; N.V. KOLESNICHENKO "Effect of pretreatment conditions on catalytic properties of zeolite catalyst in the arylalkylation of phenol by styrene"he conversion of olefins into esters via hydroboration" 000**
**CHEMICAL ABSTRACTS, Band 99, Nr. 17, 24. Oktober 1983, Seite 554, Zusammenfassungsnr. 139426f, Columbus, Ohio, US; M. ONAKA "Benzylation of acohols to benzyl ethers with**

(73) Patentinhaber: **BAYER AG, D-5090 Leverkusen 1 Bayerwerk(DE)**

(72) Erfinder: **Buysch, Hans-Josef, Dr., Brandenburger Strasse 28, D-4150 Krefeld(DE)**
Erfinder: **Klug, Günter, Dr., Ratherstrasse 49, D-4150 Krefeld(DE)**
Erfinder: **Mues, Peter, Dr., Weihers Hecke 30, D-4100 Duisberg 46(DE)**
Erfinder: **Puppe, Lothar, Dr., Am Weiher 10a, D-5093 Burscheid(DE)**

(56) Entgegenhaltungen: (Fortsetzung)
**alkali cation exchanged Y type zeolite"**
**PATENT ABSTRACTS OF JAPAN, Band 4, Nr. 54 (C-8)[536], 23. April 1980; & JP - A - 55 24145 (NIPPON SEKIYU KAGAKU) 21.02.1980**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein Verfahren zur Herstellung von p-substituierten o-Benzylphenolen durch Umsetzung von p-substituierten Phenolen mit Benzylierungsmitteln in Gegenwart von Kondensationsmitteln bei erhöhter Temperatur.

Es ist bekannt, daß bei der Benzylierung von Phenolen stets Gemische aus 2-Benzyl-, 4-Benzyl- und 2,6-Dibenzylphenolen in unterschiedlicher Zusammensetzung erhalten werden. So ist es z.B. aus J. Amer. Chem. Soc. 53, 2379 (1931) bekannt, daß bei der Kondensation von p-Kresol mit Benzylalkohol in Gegenwart von Aluminiumchlorid als Katalysator ein Produktgemisch entsteht, welches je nach dem Mengenverhältnis der Ausgangsprodukte 30 bis 36 Gew.-% an dibenzylierten Produkten enthält.

Auch mit den in der EP-A 29 333 für die Monoalkylierung von Phenolen vorgeschlagenen Katalysatoren, Mordeniten, Offretiten und Zeolith ZSM 12, d.h. Zeolithen, deren Porenvolumen zwischen dem Porenvolumen der Zeolithe des Faujasit-Typs und des Pentasil-Typs liegt, werden bei ihrer Anwendung auf die Benzylierung von Phenolen nur sehr schlechte Ausbeuten an monobenzylierten Phenolen erhalten. Das gleiche gilt für die in CA 93, (1980), S. 922 Nr. 71 184 u und für die Benzylierung von Phenol beschriebenen Katalysatoren, u.a. einen $NH_4$–Y-Katalysator. Die mit den Katalysatoren erzielten Ausbeuten an α-Methylbenzylphenol betragen höchstens 56% der Theorie und für den $NH_4$–Y-Katalysator werden sogar nur Ausbeuten bis maximal 28% der Theorie an α-Methylbenzylphenol angegeben (siehe Diagramm (a) auf S. 205 der Originalarbeit).

Weiterhin ist bekannt (Abdurasuleva et al., Zh. Org. Khim. 9, 132 (1973)), daß bei der Benzylierung von 4-Chlorphenolen mit Benzylchlorid und Kondensationskatalysatoren wie $FeCl_3$, $FeSO_4$ oder $ZnSO_4$ bis zu 18 Mol-% Dibenzylchlorphenole entstehen. Darüber hinaus wird in der Tschechischen Patentschrift 170 972 (Chemical Abstracts 89 (1978), 6109 b) die Kondensation von 4-Chlorphenol mit Benzylchlorid im Molverhältnis 4:1 in Gegenwart eines sulfonierten Styrol-divinylbenzol-copolymerisates (= stark sauren Kationenaustauschers) zu 2-Benzyl-4-chlorphenol beschrieben. Die Ausbeute an dem gewünschten Produkt beträgt zwar 83%, bezogen auf umgesetztes 4-Chlorphenol; es entsteht jedoch auch eine größere Menge an höhersiedenden Produkten; das Verhältnis von gebildetem 2-Benzyl-4-chlorphenol zu den höhersiedenden Produkten beträgt 4,16:1.

Nachteilig an den bekannten Verfahren zur Herstellung von p-substituierten o-Benzylphenolen ist, daß bei ihnen große Mengen an dibenzylierten Produkten und/oder hochsiedenden Nebenprodukten entstehen und infolgedessen die Ausbeuten an gewünschten p-substituierten o-Benzylphenolen niedrig sind. Deshalb sind die bekannten Verfahren für die industrielle Herstellung von p-substituierten o-Benzylphenolen wenig geeignet.

Es wurde nun gefunden, daß man die gewünschte Monobenzylierung p-substituierter Phenole erreichen kann, ohne die Bildung unerwünschter Nebenprodukte, wenn man die p-substituierten Phenole mit üblichen Benzylierungsmitteln bei Temperaturen von 100 bis 250°C in Gegenwart von solchen synthetischen Zeolithen des Faujasit-Typs umsetzt, die als Metallionen Natrium-, Kalium-, Caesium-, Calcium-, Zinn- oder Wasserstoffionen enthalten,

Die Erfindung betrifft daher ein Verfahren zur Herstellung von in p-Stellung substituierten mono-o-Benzylphenolen durch Umsetzung von in p-Stellung substituierten Phenolen mit üblichen Benzylierungsmitteln in Gegenwart von Kondensationsmitteln bei erhöhter Temperatur, das dadurch gekennzeichnet ist, daß man die Umsetzung bei Temperaturen von 100 bis 250°C vornimmt und als Kondensationsmittel synthetische Zeolithe des Faujasit-Typs verwendet, die als Metallionen Natrium-, Kalium-, Cassium-, Calcium-, Zinn- oder Wasserstoffionen enthalten.

Die nach dem erfindungsgemäßen Verfahren erhältlichen p-substituierten o-Benzylphenole lassen sich durch die Formel

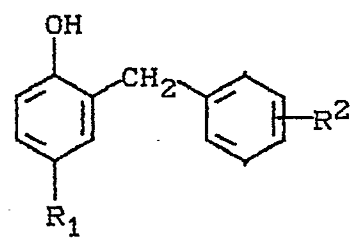 (I)

beschreiben, in der
$R^1$ Halogen, $C_1$–$C_{12}$-Alkyl, $C_1$–$C_{12}$-Alkoxy oder C1–$C_{12}$-Alkylmercapto bedeutet und
$R^2$ für Wasserstoff, Halogen, die Cyanogruppe, für $C_1$–$C_4$-Alkyl oder für eine Carb-$C_1$–$C_4$-alkoxygruppe steht.

Die im erfindungsgemäßen Verfahren als Ausgangsverbindungen zu verwendenden p-substituierten Phenole entsprechen der Formel

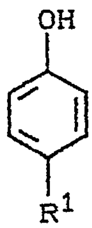

(II)

in der

$R^1$ die unter Formel (I) genannte Bedeutung hat.

Die Benzylierungsmittel entsprechen der Formel

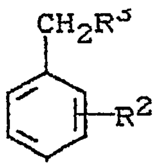

(III)

in der

$R^2$ die unter Formel (I) angegebene Bedeutung besitzt und

$R^3$ für Halogen, eine Hydroxy-, $C_1$–$C_4$-Alkoxy-, Aralkoxy- oder Aryloxy-Gruppe steht.

Als Alkylreste kommen für $R^1$ und in den für $R^1$ genannten Alkoxy- und Alkylmercapto-Gruppen solche mit 1 bis 12, bevorzugt 1 bis 4, Kohlenstoffatomen in Frage. Beispielsweise seien genannt: Methyl, Ethyl, n-Propyl, iso-Propyl, n-Butyl, iso-Butyl, sec.-Butyl, tert.-Butyl, Pentyl, Hexyl, Octyl, Decyl, Dodecyl, bevorzugt Methyl, Ethyl, n-Propyl, iso-Propyl, n-Butyl, iso-Butyl, sec.-Butyl sowie tert.-Butyl, besonders bevorzugt Methyl.

Als Halogene seien genannt: Fluor, Chlor, Brom und Iod, bevorzugt Chlor und Brom, besonders bevorzugt Chlor,

Als Carb-$C_1$–$C_4$-alkoxygruppen seien beispielsweise benannt: die Carbmethoxy-, die Carbethoxy-, Carbpropoxy- sowie Carbbutoxy-Gruppen, bevorzugt die Carbmethoxy- sowie die Carbethoxy-Gruppe.

Als Aralkoxygruppen seien solche genannt, die 1 bis 4 C-Atome im Alkylteil und 6 bis 12 C-Atome im Arylteil enthalten, z.B. die 2-Phenylethoxy- und besonders die Benzyloxy-Gruppe.

Als Aryloxyreste seien vorzugsweise gegebenenfalls substituierte Phenoxy-Reste genannt; z.B. der Phenoxy-, Tolyloxy- und der 4-Chlorphenoxy-Rest,

Für das erfindungsgemäße Verfahren können z.B. folgende p-substituierte Phenole eingesetzt werden: p-Chlorphenol, p-Kresol, Hydrochinon-monoethylether, p-Hydroxythiophenol, p-Bromphenol sowie p-Fluorphenol, bevorzugt p-Chlorphenol, p-Kresol und Hydrochinon-monomethylether, besonders bevorzugt p-Chlorphenol und p-Kresol.

Als Benzylierungsmittel der Formel (II) seien beispielsweise genannt: Benzylchlorid, Benzylalkohol, Dibenzylether und p-Chlorphenylbenzylether, bevorzugt Benzylalkohol, Dibenzylether und p-Chlorphenylbenzylether.

Erfindungsgemäß werden die Phenole und die Benzylierungsmittel im allgemeinen in einem Molverhältnis von etwa 10:1 bis 0,1:1 (Phenol:Benzylierungsmittel), bevorzugt 6:1 bis 0,2:1, besonders bevorzugt 5,1:1 bis 1,0:1 eingesetzt.

Die erfindungsgemäß als Kondensationsmittel zu verwendenden synthetischen Zeolithe des Faujasit-Typs, die als Metallionen Natrium-, Kalium-, Caesium-, Calcium-, Zinn- oder Wasserstoffatome enthalten, sind bekannt und z.B. von D.W. Breck in Zeolite Molecular Sieves, John Wiley and Sons, Inc. New York 1974, sowie von Peter A, Jacobs in Carbonionogenic Activity of Zeolites, Elsevier, Amsterdam 1977 beschrieben. Bevorzugt sind die synthetischen Zeolithe Zeolith X und Zeolith Y.

Synthetische Zeolithe des Faujasit-Typs haben die allgemeine Zusammensetzung:

$(1.0+0.2)M_{2/n}OAl_2O_3(2–6)SiO_2ZH_2O$.

wobei

M ein Metallion und

n dessen Wertigkeit bedeutet.

Als Zeolith X werden synthetische Faujasite mit einem $SiO_2/Al_2O_3$-Verhältnis von 2 bis 3, als Zeolith Y synthetische Faujasite mit $SiO_2/Al_2O_3$-Verhältnis von 3 bis 6 bezeichnet.

Die Menge der erfindungsgemäß zu verwendenden synthetischen Zeolithe des Faujasit-Typs beträgt im allgemeinen etwa 1 bis 50 Gew.-%. bevorzugt 5 bis 40 Gew.-%, bezogen auf eingesetztes Phenol.

Die Umsetzung erfolgt üblicherweise im Temperaturbereich von etwa 100 bis 250°C, vorzugsweise bei 125 bis 220°C, besonders bevorzugt bei 150 bis 200°C.

Das erfindungsgemäße Verfahren wird im allgemeinen bei Normaldruck durchgeführt. Es läßt sich jedoch auch bei erhöhtem oder vermindertem Druck durchführen, beispielsweise bei einem Druck von 0,1

bis 30 bar, bevorzugt 1 bis 10 bar, besonders bevorzugt bei 1 bis 5 bar,

Im allgemeinen beträgt die Reaktionszeit des erfindungsgemäßen Verfahrens etwa 10 Min. bis 50 Stunden, bevorzugt 30 Min, bis 30 Stunden. Die Reaktionszeit hängt aber in wesentlichen von der Aktivität und von der Menge des eingesetzten Zeoliths ab.

Die erfindungsgemäße Umsetzung kann sowohl kontinuierlich als auch diskontinuierlich durchgeführt werden.

Nach Beendigung der Umsetzung kann das Reaktionsgemisch gegebenenfalls nach Abtrennen des Kondensationsmittels in üblicher Weise durch Vakuumdestillation aufgearbeitet werden.

Die dabei im Vorlauf erhaltene Mischung aus Phenolen, Benzylierungsmitteln und geringen Anteilen des gewünschten Produktes kann nach Zugabe von Phenolen und neuem Benzylierungsmittel erneut in die Reaktion eingesetzt werden.

Nach dem erfindungsgemäßen Verfahren erhält man die o-Benzylphenole in hoher Reinheit und in Ausbeuten von über 90 % der Theorie, bezogen auf umgesetztes Phenol.

Das erfindungsgemäße Verfahren kann beispielsweise wie folgt durchgeführt werden:

In einer Apparatur, versehen mit Rührer und Rückflußkühler, wird geschmolzenes Phenol, das Kondensations- und das Benzylierungsmittel vorgelegt und dann das Reaktionsgemisch auf die geeignete Reaktionstemperatur gebracht. Es ist jedoch auch möglich, das Benzylierungsmittel nach Erreichen der Reaktionstemperatur dem Gemisch aus Phenol und Zeolith zuzugeben. Die Reaktion wird solange bei der angegebenen Reaktionstemperatur gehalten, bis das Benzylierungsmittel möglichst vollständig umgesetzt ist. Nach Abtrennen des Katalysators durch Filtration wird das Reaktionsgemisch im Vakuum destilliert, wobei man die p-substituierten o-Benzylphenole in hoher Reinheit erhält. Weiterhin ist es möglich, die Vakuumdestillation direkt, d.h. ohne vorherige Entfernung des Katalysators nach Beendigung der Reaktion durchzuführen. Bei kontinuierlicher Fahrweise wird die Reaktion zweckmäßigerweise in einem Festbettreaktor durchgeführt. Dabei beträgt die Zuführrate (LHSV = Menge Ausgangsgemisch/Menge Katalysator x h) 0.5 bis 5 $h^{-1}$, bezogen auf das Gemisch von Phenol und Benzylierungsmittel, welches gleichmäßig in einem Inertgasstrom (Stickstoff, Edelgas und/oder Wasserdampf) über eine Vorheizstrecke dem Katalysatorbett zugeführt wird. Das aus dem Reaktor austretende Reaktionsgemisch wird nach Kondensation einer Destillationskolonne zugeführt.

Die Vorteile des erfindungsgemäßen Verfahrens liegen im Vergleich zum Stand der Technik in den hohen Ausbeuten an p-substituierten o-Benzylphenolen, dem hohen Umsatz an Phenolen und dem geringen Anteil an hochsiedenden Nebenprodukten, wie Dibenzylphenolen und anderen Mehrkernaromaten.

p-Substituierte o-Benzylphenole sind bekannte Verbindungen und werden nach GB-PS 1 330 753 und US-PS 4 514 577 als Antioxidantien und Bakterizide eingesetzt.

Die nachfolgenden Beispiele sollen das erfindungsgemäße Verfahren verdeutlichen.

## Beispiele

### Beispiel 1

68.2 g (0.5 Mol) 4-Chlorphenol wurden mit 10.8 g (0.1 Mol) Benzylalkohol in Gegenwart von 6.0 g Na-Y-Zeolith innerhalb von 3 Stunden bei 200°C umgesetzt. Nach Abtrennung des Katalysators wurden 76.2 g Rohprodukt erhalten, dessen Zusammensetzung gaschromatographisch bestimmt wurde: 71.2 % 4-Chlorphenol, 25.4 % 2-Benzyl-4-chlorpohenol, 0.3 % o-Benzylphenol, 1.3 % Dibenzylether und 1.8 % höhersiedende Produkte (u.a. 2,6-Dibenzyl-4-chlorphenol). Das Rohprodukt wurde in üblicher Weise im Vakuum destilliert. Der Vorlauf enthielt 54.7 g nicht umgesetztes Chlorphenol mit geringen Anteilen an 2-Benzyl-4-chlorphenol (<1 %). Der Hauptlauf der Destillation enthielt 20.8 g 2-Benzyl-4-chlorphenol (Ausbeute 94.8 %, bezogen auf eingesetztes 4-Chlorphenol) und der Rest bestand aus einem höhersiedenden Öl.

Im folgenden werden die nachstehend aufgeführten Abkürzungen verwendet:

Z Zeolith
CP 4-Chlorphenol
BCP 2-Benzyl-4-chlorphenol
HS höhersiedende Produkte = 2,6-Dibenzyl-4-chlor- oder -4-methyl-phenol und Mehrkernaromaten
BA Benzylalkohol
BP o-Benzylphenol
DBE Dibenzylether
CPBE 4-Chlorphenyl-benzylether
PK p-Kresol
BPK 2-Benzyl-p-kresol
Rest Summe aus DBE, CPBE, BA, PKBE, o-Benzylphenol

### Beispiele 2-7

Tabelle 1 gibt die gaschromatographisch ermittelten Zusammensetzungen der Reaktionsgemische aus

CP und BA in Abhängigkeit von den verwendeten Zeolithen an. Die Molverhältnisse von CP und BA betrugen 1:1 Dazu wurden 6.5 g CP und 5.4 g BA mit 3.0 g Z 3 h lang auf 200°C erhitzt.

Tabelle 1

| Beispiel | Z | CP | BCP | HS | Rest | BCP/HS |
|---|---|---|---|---|---|---|
| 2 | H-Y | 23,9 | 52,8 | 15,0 | 8,3 | 3,52 |
| 3 | Na-Y | 17,7 | 63,1 | 15,3 | 3.9 | 4,12 |
| 4 | K-Y | 16,4 | 60,0 | 16,8 | 6,8 | 3,57 |
| 5 | Cs-Y | 27,4 | 51,9 | 15,2 | 5,5 | 3,41 |
| 6 | Na-X | 10,4 | 64,5 | 21,1 | 4,0 | 3,06 |
| 7 | Ca-Y | 32,7 | 39,3 | 10,8 | 17,2 | 3,64 |

Die obenstehende Tabelle zeigt, mit welch hoher Selektivität die Umsetzung verläuft, wenn Zeolithe des Faujasit-Typs eingesetzt werden.

So wird selbst bei einem so ungünstigen Molverhältnis von CP zu BA von 1:1 die Bildung höhermolekularer Nebenprodukte weitegehend zurückgedrängt, was besonders wirtschaftlich ist, da diese Nebenprodukte in der Regel ensorgt werden müssen. Weiterhin wird deutlich, daß Y-Zeolithe und hier besonders der Na-Y-Zeolith bezüglich Umsatz und Unterdrückung höhersiedender Produkte für die vorliegende Umsetzung die besten Ergebnisse liefert.

<u>Beispiele 8-12</u>

CP und BA wurden in unterschiedlichen Molverhältnissen 3 Stunden lang bei 200°C am Na-Y-Zeolith kondensiert. Tabelle 3 zeigt die gaschromatographisch ermittelte Zusammensetzung des Reaktionsgemisches.

Tabelle 2

| Beispiel | CP:BA | CP | BCP | HS | Rest | BCP/HS |
|---|---|---|---|---|---|---|
| 8 | 5:1 | 69,7 | 26,5 | 1,8 | 2,0 | 14,8 |
| 9 | 4:1 | 60,1 | 33,7 | 3,4 | 2,8 | 9,9 |
| 10 | 3:1 | 50,1 | 42,5 | 5,0 | 2,4 | 8,5 |
| 11 | 2:1 | 40,3 | 50,4 | 6,8 | 2,5 | 7,4 |
| 12 | 1:1 | 14,5 | 62,6 | 16,5 | 3,9 | 3,8 |

Bei der obengenannten Umsetzung wurden 1,5 g des Na-Y-Zeoliths, eingesetzt. Die Menge an CP und BA betrug insgesamt 150 mMol.

Überschüssiges CP reduziert die Bildung hochsiedender Produkte, da auf diese Weise Mehrfachkondensationen von Benzylalkohol mit bereits gebildetem BCP zu einem großen Teil vermieden werden können. So wird bei einem Molverhältnis von CP:BA von 4:1 das in der Tschechichen Patentschrift 170 972 beschriebene Verhältnis von BCP/HS = 4.26 um das Doppelte überschritten und selbst bei einem Molarverhältnis von CP/BA =2 erhält man nach dem erfindungsgemäßen Verfahren ein Verhältnis von BCP/HS = 7.4.

<u>Beispiele 13-15</u>

6.5 g CP und 5.4 g BA wurden auf die in Tabelle 4 angegebene Temperatur gebracht und dann wurden dem Gemisch 3.0 g Na-Y-Zeolith zugegeben. Tabelle 3 zeigt die gaschromatographisch ermittelte Zusammensetzung der Reaktionsgemische nach 3 Stunden Reaktionszeit.

Tabelle 3

| Beispiel | T(°C) | CP | BCP | HS | Rest | BCP/HS |
|---|---|---|---|---|---|---|
| 13 | 150 | 16,4 | 64,3 | 16,0 | 3,3 | 4,0 |
| 14 | 175 | 19,4 | 60,9 | 16,1 | 3,6 | 3,8 |
| 15 | 200 | 17,7 | 63,1 | 15,3 | 3,9 | 4,1 |

Die Tabelle 3 zeigt, daß die Reaktion auch bei 150°C noch hinreichend rasch verläuft. Im gewählten Temperaturbereich beobachtet man bei unterschiedlichen Temperaturen und bei ähnlichem Umsatz nur eine geringfügige Veränderung des Verhältnisses von BCP zu HS.

Beispiele 16-17

6.5 g CP und Benzylierungsmittel im angegebenen Molverhältnis wurden mit 3.0 g Na-Y-Zeolith 3 Stunden lang auf 200°C gehalten. Tabelle 4 zeigt die gaschromatographisch ermittelte Zusammensetzung des Reaktionsgemisches.

Tabelle 4

| Beispiel | Benzylierungsmittel | CP/Benzm. | CP | BCP | HS | Rest |
|---|---|---|---|---|---|---|
| 16 | DBE | 2 | 13,0 | 66,2 | 18,0 | 2,8 |
| 17 | CPBE | 1 | 26,1 | 57,7 | 14,5 | 1,7 |

DBE und CPBE wurden als Nebenprodukte im Vorlauf der destillativen Reinigung des Reaktionsgemisches abgetrennt. Wie Tabelle 4 zeigt, können diese Nebenprodukte durch Zugabe von CP in BCP übergeführt werden.

Beispiele 18 und 19

PK und BA wurden im Molverhältnis 4:1 gemischt und mit einem Zeolith des Faujasit-Typs (20 g/Mol PK) mehrere Stunden lang auf 200°C erhitzt. Tabelle 5 zeigt die Reaktionszeit und gaschromatographisch ermittelte Zusammensetzung der Reaktionsgemische.

Tabelle 5

| Beispiel | Zeolith | t/h | PK | BPK | HS | Rest | BPK/HS |
|---|---|---|---|---|---|---|---|
| 18 | Na-Y | 15 | 35,5 | 59,9 | 3,9 | 0,7 | 15,4 |
| 19 | Sn-Y | 5,5 | 38,5 | 56,1 | 4,0 | 1,4 | 14,0 |

Tabelle 5 zeigt, daß auch bei der Benzylierung von PK die Verwendung von Zeolithen des Faujasit-Typs zu guten Umsätzen und ausgezeichneten Selektivitäten führt. Die auf umgesetztes PK bezogene Ausbeute beträgt 94 %.

## Patentansprüche

1. Verfahren zur Herstellung von in p-Stellung substituierten mono-o-Benzylphenolen durch Umsetzung von in p-Stellung substituierten Phenolen mit üblichen Benzylierungsmitteln in Gegenwart von Kondensationsmitteln bei erhöhten Temperaturen, dadurch gekennzeichnet, daß man die Umsetzung bei Temperaturen von 100 bis 250°C vornimmt und als Kondensationsmittel synthetische Zeolithe des Faujasit-Typs verwendet, die als Metallionen Natrium-, Kalium-, Caesium-, Calcium-, Zinn- oder Wasserstoffionen enthalten.

2. Verfahren nach Anspruch 1, dadurch gekennzeichnet, daß man als synthetische Zeolithe des Faujasit-Typs Zeolith X und/oder Zeolith Y einsetzt.

3. Verfahren nach Anpsrüchen 1 oder 2, dadurch gekennzeichnet, daß die Menge der eingesetzten

Zeolithe des Faujasit-Typs 1 bis 50 Gew.-%, bezogen auf eingesetztes Phenol, beträgt.

4. Verfahren nach Anpsrüchen 1 oder 2, dadurch gekennzeichnet, daß die Menge der eingesetzten Zeolithe des Faujasit-Typs 5 bis 40 Gew.-%, bezogen auf eingesetztes Phenol, beträgt.

5. Verfahren nach einem der Anpsrüche 1, 2, 3 oder 4, dadurch gekennzeichnet, daß die in p-Stellung substituierten o-Benzylphenole der Formel

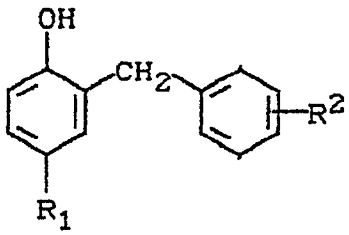

(I)

entsprechen, in der
$R^1$ Halogen, $C_1$–$C_{12}$-Alkyl, $C_1$–$C_{12}$-Alkoxy oder $C_1$–$C_{12}$-Alkylmercapto bedeutet und
$R^2$ für Wasserstoff, Halogen, die Cyanogruppe, für $C_1$–$C_4$-Alkyl oder für eine Carb-$C_1$–$C_4$-alkoxygruppe steht,

und daß diese in p-Stellung substituierten Phenole der Formel (I) durch Umsetzung von p-substituierten Phenolen der Formel

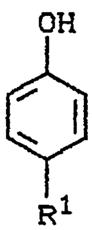

in der
$R^1$ unter Formel (I) angegebene Bedeutung hat,

mit Benzylierungsmitteln der Formel

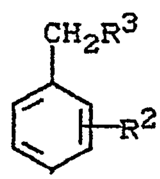

in der
$R^2$ die unter Formel (I) angegebene Bedeutung besitzt und
$R^3$ für Halogen, die Hydroxygruppe, für $C_1$–$C_4$-Alkoxy, Aralkoxy oder Aryloxy steht erhalten werden.

**Claims**

1. Process for the preparation of p-substituted mono-o-benzylphenols by reacting p-substituted phenols with conventional benzylating agents in the presence of condensing agents at elevated temperatures, characterized in that the reaction is carried out at temperatures from 100 to 250°C and the condensing agents used are synthetic zeolites of the faujasite type which contain as metal ions sodium, potassium, caesium, calcium, tin or hydrogen ions.

2. Process according to Claim 1, characterized in that zeolite X and/or zeolite Y are employed as synthetic zeolites of the faujasite type.

3. Process according to Claims 1 or 2, characterized in that the amount of faujasite-type zeolites employed is 1 to 50% by weight, relative to phenol employed.

4. Process according to Claims 1 or 2, characterized in that the amount of faujasite-type zeolites employed is 5 to 40% by weight, relative to phenol employed.

5. Process according to one of Claims 1, 2, 3 or 4, characterized in that the p-substituted o-benzylphenols correspond to the formula

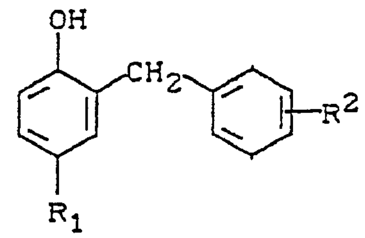 (I)

in which

$R^1$ denotes halogen, $C_1$–$C_{12}$-alkyl, $C_1$–$C_{12}$-alkoxy or $C_1$–$C_{12}$-alkylmercapto, and

$R^2$ represents hydrogen, halogen, the cyano group, $C_1$–$C_4$-alkyl or a carb-$C_1$–$C_4$-alkoxy group, and in that these p-substituted phenols of the formula (I) are obtained by reacting p-substituted phenols of the formula

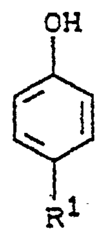

in which

$R^1$ has the meaning given under formula (I), with benzylating agents of the formula

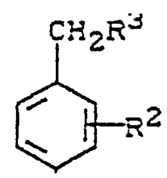

in which

$R^2$ has the meaning given under formula (I) and

$R^3$ represents halogen, the hydroxyl group, $C_1$–$C_4$- alkoxy, aralkoxy or aryloxy.

**Revendications**

1. Procédé de production de mono-o-benzyl-phénols substitués en position para, par réaction de phénols substitués en position para avec des agents classiques de benzylation en présence d'agents de condensation à des températures élevées, caractérisé en ce qu'on conduit la réaction à des températures de 100 à 250°C et on utilise comme agents de condensation des zéolites synthétiques du type de la faujasite, qui contiennent comme ions métalliques des ions sodium, potassium, césium, calcium, étain ou des ions hydrogènes.
2. Procédé suivant la revendication 1, caractérisé en ce qu'on utilise comme zéolites synthétiques du type de la faujasite, la zéolite X et/ou la zéolite Y.
3. Procédé suivant les revendications 1 ou 2, caractérisé en ce que la quantité de zéolite utilisée du type faujasite s'élève à 1–50% en poids par rapport au phénol utilisé.
4. Procédé suivant les revendications 1 ou 2, caractérisé en ce que la quantité de zéolites utilisées du type de la faujasite s'élève à 5–40% en poids par rapport au phénol utilisé.
5. Procédé suivant l'une des revendications 1, 2, 3 ou 4, caractérisé en ce que les o-benzylphénols substitués en position para répondent à la formule

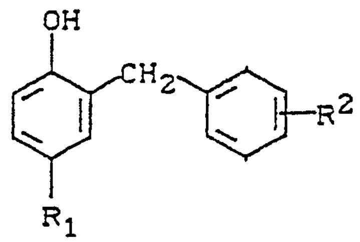

(I)

dans laquelle
$R^1$ désigne un halogène, un groupe alkyle en $C_1$ à $C_{12}$, un groupe alkoxy en $C_1$ à $C_{12}$ ou un groupe alkylmercapto en $C_1$ à $C_{12}$ et
$R^2$ désigne l'hydrogène, un halogène, le groupe cyano, un groupe alkyle en $C_1$ à $C_4$ ou un groupe carb(alkoxy en $C_1$ à $C_4$),
et en ce que ces phénols de formule (I) substitués en position para sont obtenus par réaction de phénols substitués en position para de formule

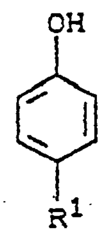

dans laquelle
$R^1$ a la définition indiquée pour la formule (I), avec des agents de benzylation de formule

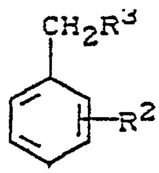

dans laquelle
$R^2$ a la définition indiquée pour la formule (I) et
$R^3$ désigne un halogène, le groupe hydroxy, un groupe alkoxy en $C_1$ à $C_4$, aralkoxy ou aryloxy.